# EUROPEAN PATENT APPLICATION

(11) **EP 0 985 382 A1**
(43) Date of publication of application: **15.03.2000**
(21) Application number: 99306755.2
(22) Date of filing: 25.08.1999
(51) Int. Cl.: A61B 17/06, D04B 3/02

(54) **Surgical double-tipped needle**

(30) Priority: 25.08.1998 KR 9815941; 30.04.1999 KR 9915727
(71) Applicant: Baek, Seung Jun, Seocho-gu, Seoul (KR)
(72) Inventor: Baek, Seung Jun, Seocho-gu, Seoul (KR)
(74) Representative: Mounteney, Simon James

(57) **Abstract**

In the present invention, a surgical operation double-tipped needle (10) is disclosed. The surgical operation needle includes at least one needle eye (12), through which a suture is inserted, formed at an intermediate portion of the double-tipped needle, a pair of guide grooves (13) formed at both sides of the needle eyes and each having a certain length, and gauges formed at both ends of the double-tipped needle, so that it is possible to check a penetration depth of the double-tipped needle when the double-tipped needle is penetrated into the skin wherein there a circular operation double-tipped needle has a certain curvature. In the surgical operation double-tipped needle (10), since another double-tipped needle is penetrated into the skin via the guide grooves, it is possible to minimize the sutured portion.

## Description

### BACKGROUND OF THE INVENTION

### 1. Field of the Invention

The present invention relates to a surgical operation needle, and in particular to a surgical operation needle in which a guide groove is formed in the length direction of a body of a surgical operation needle and gauges provided at both ends of the same.

### 2. Description of the Background Art

Generally, a surgical operation needle is used for suturing cut skin and for a plastic operation such as a double eyelid operation, etc. The above-described surgical operation needle is fabricated in various shapes.

For example, the surgical operation needle has a certain cross section such as a triangle cross section, a circular cross section, etc. The needle body and shape are variously formed. The surgical operation needle has a semi-circular body, a circular shape body having a length of about 3/8 with respect to the entire circular length, and a straight shape body.

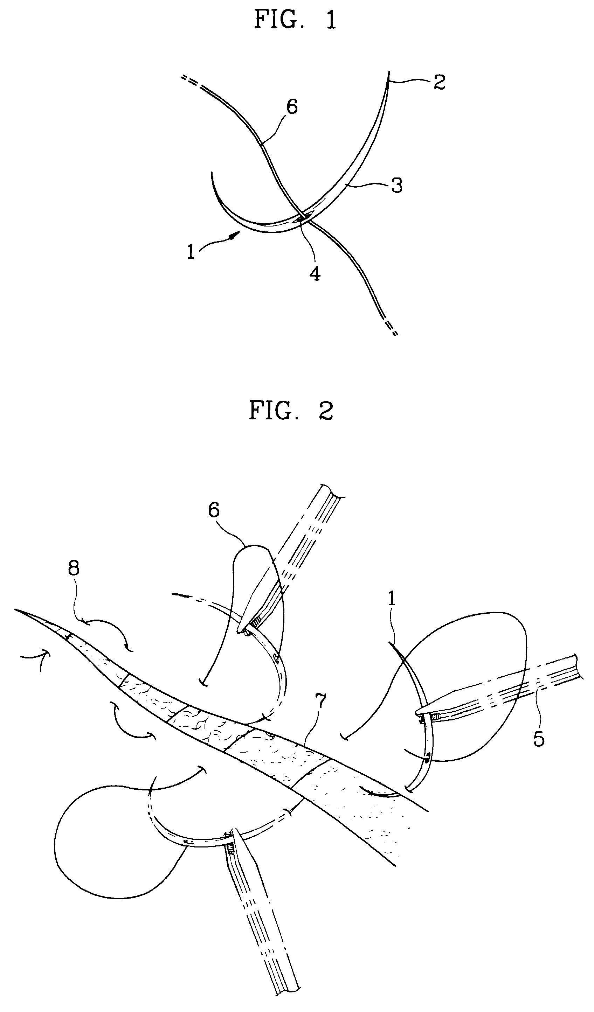

Figure 1 is a perspective view illustrating a conventional surgical operation needle, and Figure 2 is a view illustrating a suturing operation which is performed using a conventional surgical operation needle.

As shown therein, a surgical operation double-tipped needle 1 has a tip 2 at both ends of the needle 1, and a body 3 formed in a certain circular shape. A needle eye 4 is formed at an intermediate portion of the double-tipped needle 1. An operation suture 6 passes through the needle eye 4. Therefore, when performing a certain operation, the suture 6 is inserted into the needle eye 4.

When performing an operation using the double-tipped needle 1, the double-tipped needle 1 is held using a pincette 5, and a suturing portion is stitched using the double-tipped needle 1 in a zig-zag direction for thereby implementing a suturing operation.

However, when suturing using the double-tipped needle 1, a suture is exposed from a skin, so that a certain scar may be formed. In addition, when forming a knot of a suture after a suturing operation is finished, a skin is cut for thereby forming a cut portion 8.

Therefore, the conventional surgical operation double-tipped needle is not generally used for a double eyelid operation due to the above-described problem. In addition, when suturing a certain portion at a corium, a certain portion of the corium is stitched using a double-tipped needle depending on a stitching depth by a doctor's sensory ability. Therefore, it is impossible to implement an accurate and stable surgical operation in the conventional art.

### SUMMARY OF THE INVENTION

Accordingly, it is an object of the present invention to provide a surgical operation needle which makes it possible to minimize a scar of a sutured portion without cutting and accurate suture a certain portion using a gauge capable of measuring a suturing depth.

To achieve the above object, there is provided a surgical operation needle which includes at least one needle eye, through which a suture is inserted, formed at an intermediate portion of the double-tipped needle, a pair of guide grooves formed at both sides of the needle eyes and each having a certain length, and gauges formed at both ends of the double-tipped needle, so that it is possible to check a penetration depth of the double-tipped needle when the double-tipped needle is penetrated into the skin wherein there a circular operation double-tipped needle has a certain curvature.

In the present invention, since a pair of guide grooves are formed at a certain depth, another double-tipped needle is penetrated into the skin via the guide grooves, so that the sutured portion is minimized. The guide grooves may be formed on the upper and lower surfaces of the double-tipped needle.

Additional advantages, objects and features of the invention will become more apparent from the description which follows.

### BRIEF DESCRIPTION OF THE DRAWINGS

The present invention will become more fully understood from the detailed description given herein below and the accompanying drawings which are given by way of illustration only, and thus are not limitative of the present invention, and wherein:
Figure 1 is a perspective view illustrating a state that a suture passes through a conventional surgical operation double-tipped needle;
Figure 2 is a view illustrating a process that a suturing operation is performed using a conventional surgical operation double-tipped needle;
Figure 3 is a perspective view illustrating a state that a suture passes through a surgical operation double-tipped needle according to the present invention;
Figure 4 is a cross-sectional view illustrating a guide groove formed in a surgical operation double-tipped needle according to the present invention;
Figure 5 is a cross-sectional view taken along line A-A of Figure 4; and
Figures 6A through 6K are views illustrating a process that a double eyelid operation is performed using a surgical operation double-tipped needle according to the present invention.

### DETAILED DESCRIPTION OF THE PREFERRED EMBODIMENTS

The embodiments of the present invention will be explained with reference to the accompanying drawings.

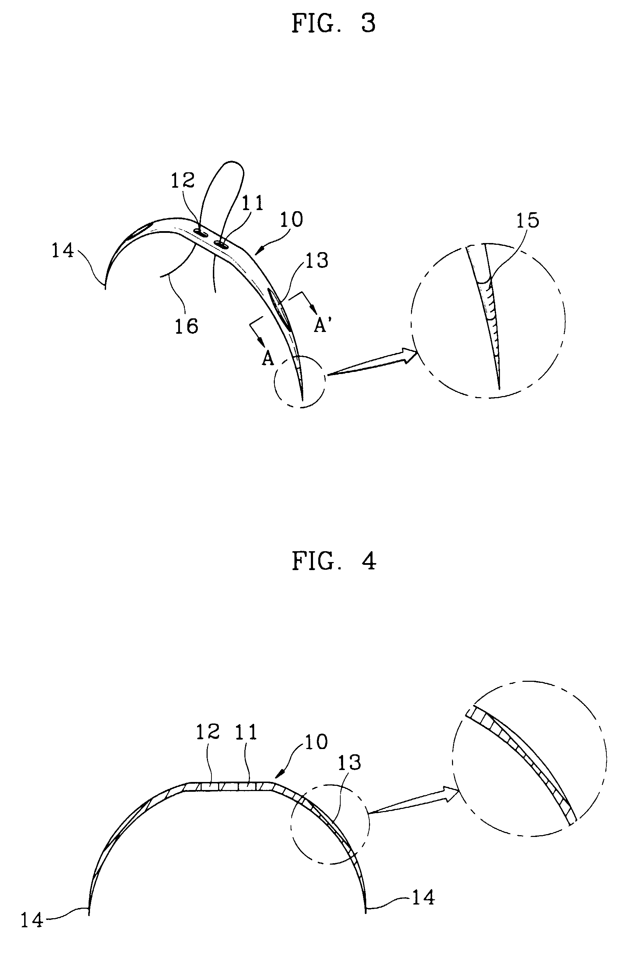

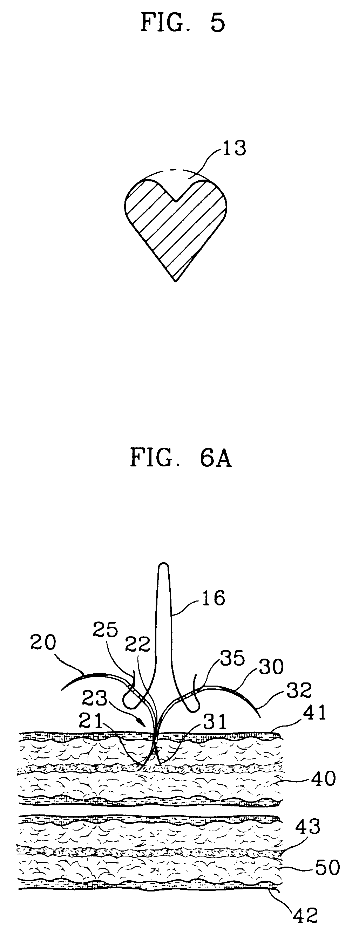

Figure 3 is a perspective view illustrating a surgical operation double-tipped needle according to the present invention. Figure 4 is a cross-sectional view illustrating a surgical operation double-tipped needle. Figure 5 is a cross-sectional view taken along line A-A of Figure 4.

As shown therein, a surgical operation double needle 10 has tips 14 formed at both ends thereof and a circular body 11 having a certain curvature. Two needle eyes 12 through which a suture passes are formed at an intermediate portion of the body 11 of the double-tipped needle 10. One end of the suture 16 is inserted into one of two needle eyes 12 and then is inserted into the other of two needle eyes, so that the suture 16 is stably engaged with the double-tipped needle 10.

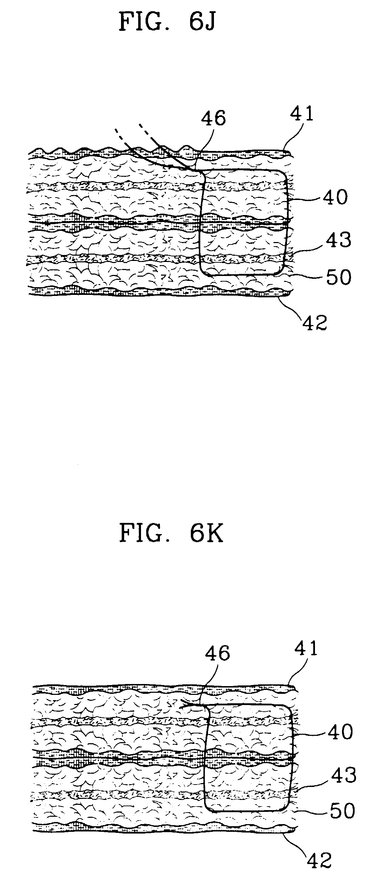

Guide grooves 13 are formed at a certain depth at both sides of two needle eyes 12 in a length direction. Therefore, when forming a certain through portion 23, as shown in Figure 6A, of the circular needle 10 via a certain portion of a skin, since the double-tipped needle 10 passes through the through portion 23 via the guide grooves 13, so that it is possible to prevent an over expanding of the through portion 23. Here, the guide grooves 13 may be formed on an upper side and lower side of the double-tipped needle 10.

In addition, a scale is formed on outer surfaces of the tips 14, respectively, for thereby forming gauges 15 capable of measuring a suturing depth. Preferably, a scale of 1mm is formed by a length of 1.5cm. The gauge 15 is formed in an embossing method, an engraving method, or an inlaying method. Therefore, it is possible to measure a depth when the double-tipped needle 10 is inserted into a suturing portion ofa skin, so that a doctor accurately sutures a certain portion of a corium 43, as shown in Figure 6A, using the double-tipped needle 10.

The operation of the double-tipped needle according to the present invention will be explained with reference to the accompanying drawings.

As shown in Figure 6A, when performing a double eyelid operation using first and second double-tipped needles 20 and 30, a suture 16 is inserted into needle eyes 25 and 35 of the first and second double-tipped needles 20 and 30. Thereafter, the first double-tipped needle 20 is penetrated into a certain portion of an upper skin 40, so that a guide groove 26 is positioned on an outer surface 41 of the upper skin 40.

At this time, a through portion 23 having the same diameter as the diameter ofthe first double-tipped needle 20 is formed at the upper skin 40 into which the first double-tipped needle 20 is penetrated. In this state, the second double-tipped needle 30 passes through the through portion 23 via the guide groove 26 of the first double-tipped needle 20 and is penetrated into the upper skin 40. Therefore, it is possible to prevent the through portion 23 from being expanded by a certain length. In this state, the second double-tipped needle 30 is continuously penetrated into the upper skin 40 and passes through the upper and lower skins 40 and 50.

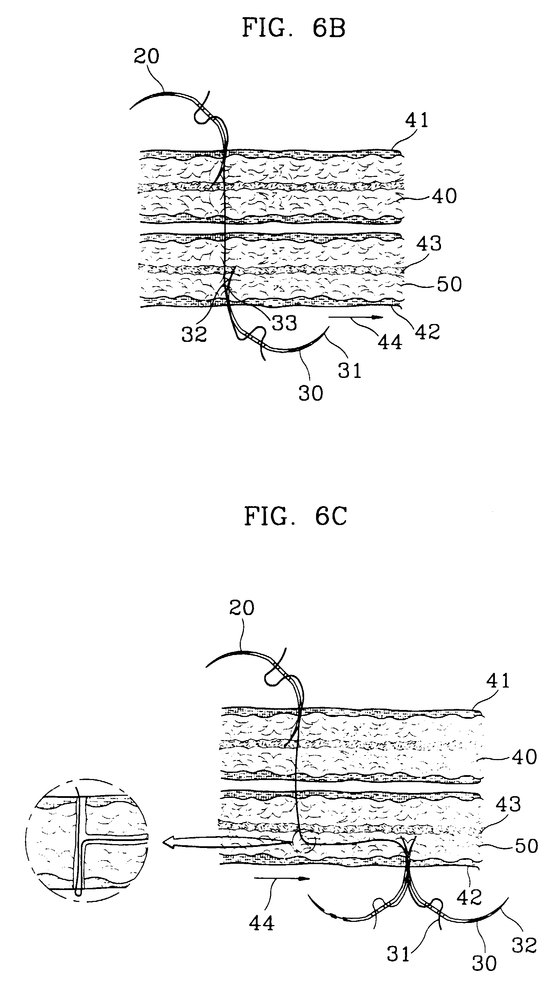

As shown in Figure 6B, one end 31 of the second double-tipped needle 30 comes out beyond the surface 42 of the lower skin 50. At this time, the other end 32 of the second double-tipped needle 30 is positioned in the lower skin 50.

In addition, a doctor checks the scales of the gauge 33 formed at the other end 32 of the second double-tipped needle 30, so that it is possible to check the position of the other end 32. In addition, the other end 32 may be penetrated in a certain direction 44 in the lower skin 50 by pushing one end 31 of the second double-tipped needle 30.

As shown in Figure 6C, the other end 32 of the second double-tipped needle 30 comes out from the surface 42 of the lower skin 50. At this time, one end of the second double-tipped needle 30 is positioned in the lower skin 50. In this state, the second double-tipped needle 30 passes through the lower and upper skins 50 and 40 by upwardly penetrating the second double-tipped needle 30.

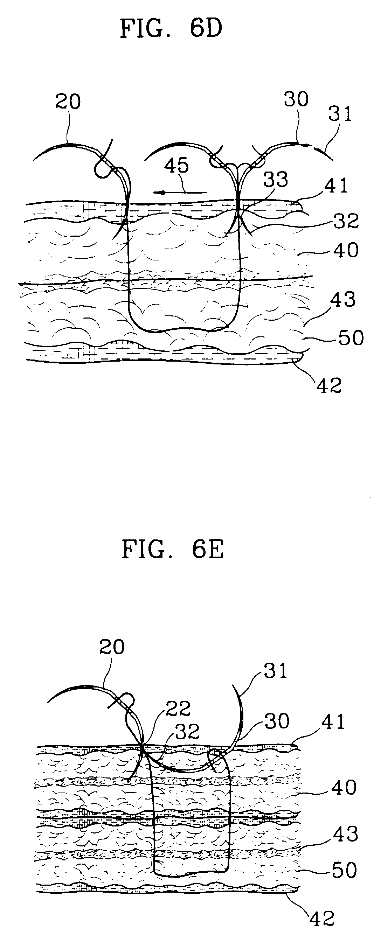

As shown in Figure 6D, the second double-tipped needle 30 is penetrated until one end 31 of the second double-tipped needle 30 comes out from the surface 41 of the upper skin 40. At this time, the other end of the second double-tipped needle 30 is positioned in the upper skin 40, and a doctor checks the scales of the gauge 33, so that it is possible to check the position of the other end 32.

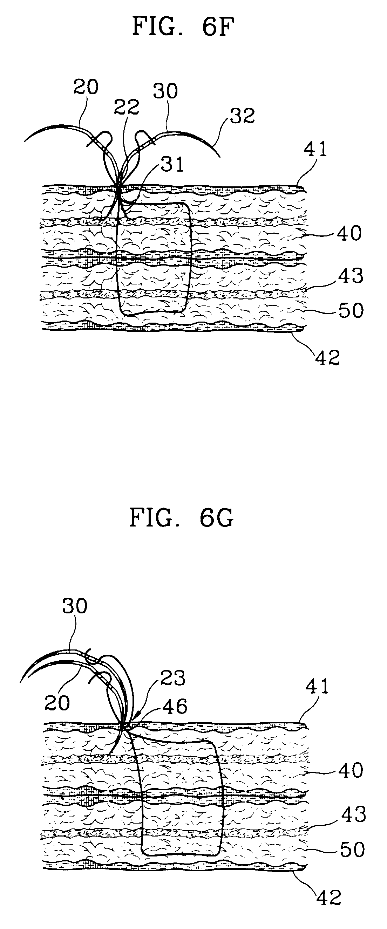

As shown in Figure 6E, the other end 32 of the second double-tipped needle 30 is penetrated in a lateral direction 45 in the upper skin 30 by penetrating one end 31 of the second double-tipped needle 30. In addition, in order to form a knot 46 as shown in Figure 6G, the other end 32 of the second double tip blade 30 is penetrated into the guide groove 22 of the first double-tipped needle 20.

As shown in Figures 6F and 6G, the other end 32 of the second double-tipped needle 30 comes out from the upper skin 40 via the guide groove 22 of the first double-tipped needle 20. In addition, the first and second double-tipped needles 20 and 30 become crossed, so that the suture 16 is crossingly formed for thereby forming the knot 46. At this time, since the knot 46 contacts with an outer surface of the skin, a certain inflammation may be caused due to bacteria. Therefore, in order to prevent the above-described problem, the knot 46 is formed at a certain depth in the skin.

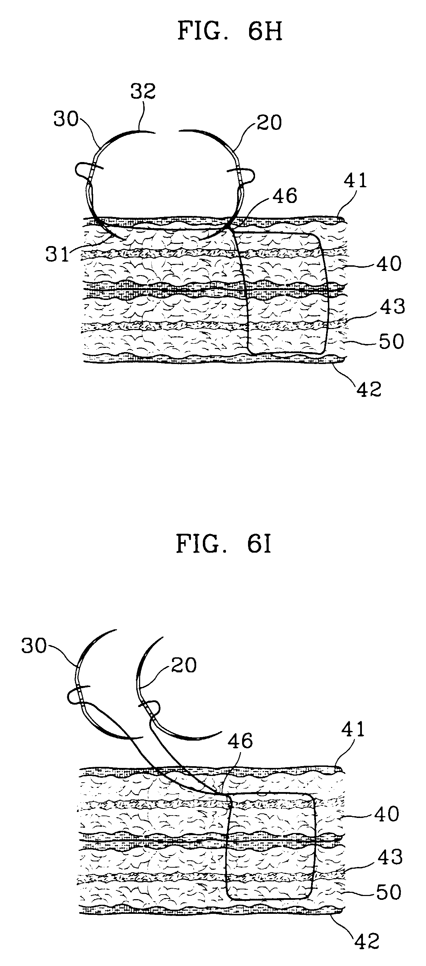

As shown in Figures 6H, 6I and 6J, one end 31 of the second double-tipped needle 30 is pushed, so that the other end of the same is penetrated in a lateral direction in the upper skin 40. In addition, the other end of the second double-tipped needle 30 comes out from the surface 41 of the upper skin 40, and the first double-tipped needle 20 comes out from the surface 41 of the upper skin 40. At this time, a certain wrinkle is formed on an outer surface of the skin at a certain interval. The suture 16 is cut for thereby finishing an operation.

As shown in figure 6K, the suture 16 is cut, and the wrinkle is recovered, and the suture 16 is embedded in the skin at a certain depth. Therefore, any scar is not formed on the surface of the skin.

Therefore, a double eyelid is successfully performed by the first and second double-tipped needles.

As described above, in the present invention, it is possible to minimize a sutured portion by penetrating a double-tipped needle into a skin via a guide groove without cutting the skin for thereby preventing a scar formation.

In addition, in the present invention, since it is possible to measure the depth of a double-tipped needle using gauges, it is possible to accurate suture a certain portion by selecting a double tip needed depending on the kinds of operation.

Since a suturing operation is performed based on a minimum through portion without cutting the skin, a small amount of anesthetic is decreased, so that it is possible to prevent any shock to thus enhance the health of a patient.

Although the preferred embodiment of the present invention have been disclosed for illustrative purposes, those skilled in the art will appreciate that various modifications, additions and substitutions are possible, without departing from the scope and spirit of the invention as recited in the accompanying claims.

## Claims

1. A curved double-tipped needle (10, 20, 30) suitable for surgical use, comprising:
at least one needle eye (12) for receiving a suture (16), formed at an intermediate point between said two tips (14 or 21, 22 or 31, 32) and characterised in that there are a plurality of guide grooves (13) extending along at least a portion of said needle's length, and running generally parallel to the needle (10, 20, 30), a guide groove (13) being between the eye and each tip (14 or 21, 22 or 31, 32).

2. The needle (10, 20, 30) of Claim 1, wherein said grooves (13) are spaced from said eye (12).

3. The needle (10, 20, 30) of Claim 1 or 2, wherein a gauge (15) is formed at each end of the double-tipped needle (10, 20, 30), suitable for allowing the penetration depth of said needle (10, 20, 30) into tissue to be monitored during use.

4. The needle (10, 20, 30) of Claim 3, wherein the gauge (15) is formed of at least one calibrated line, generally perpendicular to the length of the needle (10, 20, 30).

5. The needle (10, 20, 30) of any of the preceding Claims, wherein the said grooves (13) are formed so as to allow a second needle to operably slide in said grooves (13), for minimising the penetrated area of surface tissue during a suturing operation.

6. The needle (10, 20, 30) of any of the preceding Claims, wherein said guide grooves (13) are formed on opposite sides of said needle (10, 20, 30).

7. The needle (10, 20, 30) of any of the preceding Claims, wherein the said needle (10, 20, 30) substantially comprises an arc of a circle.

8. The needle (10, 20, 30) of any of the preceding Claims, wherein the said needle (10, 20, 30) comprises two arcs separated by a straight portion.

9. The needle (10, 20, 30) of any of the preceding Claims, wherein the said needle (10, 20, 30) has two needle eyes (12).

10. A method of surgery using the needle (10, 20, 30) of any of the preceding Claims.
